# FASCICULE DE BREVET EUROPEEN

(11) **EP 1 157 039 B1**
(45) Date de publication et mention de la délivrance du brevet: **17.11.2004**
(21) Numéro de dépôt: 00907750.4
(22) Date de dépôt: 01.03.2000
(51) Int. Cl.: C07K 14/78, C07K 1/107, A61L 24/10, A61L 27/24, A61L 15/32

(54) **PEPTIDES COLLAGENIQUES MODIFIES PAR GREFFAGE DE FONCTIONS MERCAPTO, L'UN DE LEURS PROCEDES D'OBTENTION ET LEURS APPLICATIONS COMME BIOMATERIAUX**
DURCH PFROPFUNG VON MERKAPTOGRUPPEN MODIFIZIERTE KOLLAGENPEPTIDE, EIN VERFAHREN ZU IHRER HERSTELLUNG UND IHRE ANWENDUNG ALS BIOMATERIALIEN
COLLAGEN PEPTIDES MODIFIED BY GRAFTING MERCAPTO FUNCTIONS, METHOD FOR THE PRODUCTION THEREOF AND USES THEREOF AS BIOMATERIALS

(30) Priorité: 02.03.1999 FR 9902727
(43) Date de publication de la demande: 28.11.2001
(73) Titulaire: FLAMEL TECHNOLOGIES, 69693 Venissieux Cédex (FR)
(72) Inventeur: NICOLAS, Florence, F-69740 Genas (FR); BRYSON, Nathan, F-69390 Millery (FR)
(74) Mandataire: Fleurance, Raphael
(86) Numéro de dépôt international: PCT/FR2000/000513
(87) Numéro de publication internationale: WO 2000/052052

(56) Documents cités:
- EP-A- 0 890 663
- WO-A-90/05755
- FR-A- 2 692 582
- DATABASE CHEMABS [en ligne] CHEMICAL ABSTRACTS SERVICE, COLUMBUS, OHIO, US; STN, CAPLUS accession no. 1979:422431, XP002125733 & Y. CHONAN ET AL: HIKAKU KAGAKU, vol. 24, no. 3, 1978, pages 140-147,
- C. LIN ET AL: BIOCHIM. BIOPHYS. ACTA, vol. 1038, no. 3, 1990, pages 382-385, XP000864311

## Description

La présente invention concerne de nouveaux peptides collagéniques chimiquement modifiés par greffage de fonctions thiols libres ou substituées, portées par des restes mercaptoaminés. Lorsque les peptides collagéniques comportent des fonctions thiol, ils ont la propriété d'être réticulables par oxydation et conduisent à un dérivé de collagène réticulé par des ponts disulfures.

L'invention vise également un procédé de préparation de ces nouveaux dérivés de collagène qui sont sous forme réticulable, sous forme d'un précurseur du dérivé réticulable ou bien encore sous forme réticulée.

L'invention a également trait aux applications de ces nouveaux peptides collagéniques à titre de biomatériaux utiles comme matières premières pour la fabrication de produits médicaux, chirurgicaux ou cosmétiques, tels que les tissus ou les organes artificiels, la peau artificielle, les prothèses ou implants osseux, ligamentaires, cardio-vasculaires, intra-oculaires, intrapéritonéaux ... ou bien encore les systèmes de bioencapsulation (implants, microsphères, microcapsules) permettant la libération prolongée et contrôlée de principes actifs in vivo. Les accessoires médicaux tels que les fils de suture ainsi que les revêtements de biocompatibilisation d'articles médicaux implantables, constituent d'autres illustrations des possibilités d'application des nouveaux biomatériaux selon l'invention.
Au sens de la présente invention, le terme peptide collagénique désigne notamment le collagène avec ou sans télopeptides, le collagène dénaturé ainsi que la gélatine.
On trouve sur le marché différentes qualités commerciales de collagène, avec ou sans télopeptides. Ces collagènes commerciaux peuvent être d'origine humaine ou animale.
Le collagène est une protéine connue, présente à tous les niveaux de l'organisation des tissus animaux : il s'agit de la principale protéine de la peau et des tissus conjonctifs. Par nature, il possède des caractéristiques biochimiques et physico-chimiques relativement bien adaptées pour des utilisations en tant que biomatériaux. Ces caractéristiques sont, notamment : bonne biocompatibilité, biodégradabilité, caractère hémostatique ...

Cependant, force est de constater que les articles médicaux, chirurgicaux ou cosmétiques implantables à base de collagène souffrent de certaines lacunes. Leurs caractéristiques mécaniques sont faibles et rendent leur manipulation délicate, voire les rendent inutilisables pour certaines applications. De plus, leur biodégradation peut être trop rapide lorsque les implants doivent exercer des fonctions palliatives et/ou curatives pour de longues périodes. Pour améliorer les caractéristiques mécaniques et de biodégradation des implants à base de collagène, il s'avère nécessaire de le modifier chimiquement, en particulier de le réticuler.

Pour modifier, en particulier réticuler, des peptides collagéniques, on utilise les fonctions réactives présentes sur les chaînes latérales de certains acides aminés du collagène, à savoir :
- les fonctions amines des résidus lysine, représentant en nombre 3 % des acides aminés,
- les fonctions acides carboxyliques des acides aspartiques et glutamiques, représentant en nombre 9 à 12 % des acides aminés,
- les fonctions alcool des résidus sérine, thréonine et hydroxyproline, représentant en nombre 14 % des acides aminés.

C'est ainsi que sont apparus quatre grands types de techniques de réticulation artificielle de ce peptide collagénique.
**1.** Création d'un réseau par liaisons covalentes entre les molécules de collagène, par irradiation, déshydratation poussée . Cette réticulation est obtenue sans fonctionnalisation chimique du collagène.
**2.** Activation des groupements naturels du collagène, pour introduire la possibilité d'autoréticulation, par exemple par oxydation (périodate) ou par activation fonctionnelle (activation des acides par des carbodiimides, sous forme d'azide ... qui réagissent avec les amines).
**3.** Réticulation à l'aide d'agents chimiques pontants bi- ou poly-fonctionnels (aldéhydes, composés dicarboxyliques, diamines, diisocyanates, disulfonyl-chlorures, polyéthylèneglycol difonctionnalisé).
**4.** Copolymérisation par liaisons covalentes du collagène avec un autre polymère (polyacrylique, copolyacrylonitrile-styrène, polyuréthane, polyalcool, silicone).
Une variante de la réticulation de type 3. par pontage peut consister à mettre en oeuvre des dérivés difonctionnels contenant des groupements disulfure. Cette variante est celle à laquelle on s'intéresse dans le cadre de l'invention. Ladite variante a donné lieu, dans l'art antérieur, à diverses propositions techniques, qui vont être présentées ci-après.

L'article de F. SCHADE & H. ZAHN [Einbau von cystin-brücken in Kollagen, Angew. Chem., 74, 904, 1962], décrit la fonctionnalisation de collagène à l'aide d'un dérivé de la cystine, par formation de liaisons amides entre, d'une part, les motifs NH₂ libres des résidus lysine de la chaîne collagénique et, d'autre part, les motifs carboxyles du dérivé de cystine, préalablement activés par estérification à l'aide de nitrophénol. La réduction des ponts disulfure des dérivés de cystine greffés conduit à un matériau thiolisé réticulable par oxydation. Dans la mesure où seuls les résidus lysine du collagène sont fonctionnalisés, le taux maximal de fonctionnalisation, directement proportionnel au niveau de réticulation, est au maximum de 3 % en nombre.

La demande de brevet européen EP 0 049 469 divulgue la fonctionnalisation de collagène soluble extrait de tendons à l'aide de N-acétyl homocystéine thiolactone. Il s'agit là encore d'une réaction entre les motifs carboxyles de l'agent de fonctionnalisation et les motifs amine des résidus lysine du collagène. Le taux maximum de fonctions thiols greffées est donc là aussi au maximum de 3%.

Pour obtenir de nouveaux dérivés collagéniques thiolés et/ou pour augmenter les taux de greffage de fonctions thiols sur le collagène et par suite le niveau de réticulation, la demanderesse a proposé, en son temps, trois nouvelles voies de fonctionnalisation chimique du collagène par des groupements porteurs de fonctions thiols ou des précurseurs de ceux-ci.

La première voie est décrite dans le brevet français FR 2 692 582 qui concerne un collagène greffé avec des dérivés thiolés (cystéine, homocystéine, cystéamine) :
- par l'intermédiaire d'une rotule succinique dont l'une des extrémités carboxyle a réagi avec des motifs amine des résidus lysine et avec certains motifs alcool des résidus sérine thréonine et hydroxyproline du collagène et dont l'autre extrémité carboxyle a réagi avec le motif amine du dérivé thiolé;
- et éventuellement directement sans rotule sur les fonctions carboxyle des acides aspartiques et glutamiques du collagène.
On peut ainsi atteindre jusqu'à 29 % de fonctionnalisation des acides aminés du collagène.
Les fonctions mercaptoaminées - c'est-à-dire les dérivés thiolés - décrites dans ce brevet français, sont fixées directement ou indirectement sur les fonctions NH₂, OH et COOH libres du collagène. Ce brevet ne divulgue pas de peptide collagénique dont les motifs OH et NH₂ sont fonctionnalisés par d'autres fonctions que des fonctions mercaptoaminées.

La deuxième voie est donnée dans le brevet FR 2 699 184 ayant trait à un collagène greffé avec des dérivés thiolés (cystéine, homocystéine) fixés directement sur les motifs amine des résidus lysine et certains motifs alcool des résidus sérine, thréonine et hydroxyproline. Conformément à l'invention décrite par ce brevet, l'agent de fonctionnalisation (e.g. cystine) précurseur du dérivé thiolé greffé sur le collagène comprend une fonction carboxyle activée, réagissant avec les NH₂ des lysines pour former des amides et avec les OH des sérines, thréonines et hydroxyprolines pour former des esters. Cet agent de fonctionnalisation comprend également une fonction amine protégée, qui ne peut pas réagir avec les carboxyles des acides aspartique et glutamique de la chaîne collagénique. Le taux maximum de greffage que l'on peut atteindre par cette méthode est de 17 %.

Une troisième voie de modification chimique du collagène mise au point par la demanderesse pour apporter une fonctionnalité réticulante à un tel polymère, est décrite dans le brevet français FR 2 723 957. Ce brevet divulgue un collagène greffé sur les motifs amine libres de ses résidus lysine, par un dérivé thiolé constitué par de la cystéine ou de l'homocystéine dont les fonctions amine et thiol sont protégées par un seul et même groupement protecteur, l'ensemble formant un motif thiazolidine. L'acide carboxylique du dérivé thiazolidine est activé pour pouvoir réagir avec les fonctions amines des résidus lysine. En conséquence, le taux de greffage est ici d'au maximum 3 %. Les fonctions carboxyliques libres des acides glutamiques et aspartiques de la chaîne collagénique ne sont pas substituées dans le collagène selon ce brevet.

Les collagènes selon ces trois brevets français permettent la préparation d'articles médicaux (gels, feutres, films ...) présentant des niveaux de réticulation, c'esi-à-dire des caractéristiques mécaniques et de biodégradation, intéressantes. Ils restent cependant perfectibles.

On connaît par ailleurs des collagènes substitués par des groupements qui ne sont pas des fonctions de réticulation et qui sont destinés à procurer au collagène d'autres propriétés, par exemple en modulant ses caractéristiques de solubilité et/ou ses caractéristiques rhéologiques et/ou ses caractéristiques biologiques. C'est ainsi que la demande de brevet PCT WO 90/05755 décrit un collagène, dont les amines des résidus lysine qu'il comprend, sont substituées par une chaîne polymère hydrophile synthétique et plus particulièrement par du monométhylPolyEthylèneGlycol. Ce collagène-PEG est présenté comme ayant une immunogénicité réduite et des propriétés mécaniques d'élasticité et de malléabilité améliorées.
La demande de brevet PCT WO 94/01483 divulgue quant à elle un matériau polymère conjugué biocompatible, biologiquement inerte, formé par un polymère naturel tel que le collagène, lié par une liaison éther à un polymère hydrophile synthétique tel que le polyéthylèneglycol (PEG).

Les collagènes modifiés selon l'art antérieur ne procurent pas toutes les satisfactions souhaitables, quand à leurs propriétés mécaniques, leurs cinétiques de dégradation *in vivo* et leurs caractéristiques biologiques. Par ailleurs, les collagènes connus et modifiés par des fonctions thiols libres ou substituées restent encore perfectibles, en ce qui concerne le contrôle, au travers du taux de réticulation, de leurs caractéristiques mécaniques et biologiques.
Enfin, les formes réticulables des collagènes modifiés connus gagneraient à posséder des propriétés de solubilité dans une large gamme de pH, de façon à faciliter leur mise en oeuvre et ce sans que cela ne porte préjudice à leur niveau de réticulation.

Dans cet état de l'art, l'un des objectifs essentiels de l'invention est de fournir de nouveaux collagènes modifiés par greffage de fonctions thiols, libres ou substituées, ces nouveaux collagènes se devant d'être aptes à réticuler de manière suffisante et contrôlée, par formation de ponts disulfure intercaténaires.

Un autre objectif essentiel de l'invention est de fournir de nouveaux collagènes modifiés par greffage de fonctions thiols et caractérisés par de forts taux de greffage coexistant avec une bonne solubilité dans une large gamme de pH.

Un autre objectif essentiel de l'invention est de fournir de nouveaux collagènes modifiés par greffage de fonctions thiols et faciles à mettre en oeuvre et à manipuler industriellement.

Un autre objectif essentiel de l'invention est de fournir de nouveaux collagènes modifiés par greffage de fonctions thiols, dans lesquels les fonctions réactives ne sont pas toutes mobilisées par la réticulation, de manière à permettre le greffage de fonctionnalités non-réticulantes.

Un autre objectif essentiel de l'invention est de fournir de nouveaux collagènes réticulables ou des précurseurs de collagène réticulable mercapto-fonctionnalisés et transformables en gels, films ou feutres (e. g.) de densité de réticulation (donc de résistance mécanique et de biodégradation) modulables à l'avance, de façon à fournir un éventail varié de matières premières utilisables dans de nombreuses applications comme biomatériaux..

Un autre objectif essentiel de l'invention est de fournir un procédé simple pour la préparation d'un peptide collagénique modifié par greffage de fonctions thiols libres ou substituées et portées par des restes mercaptoaminés.

Les inventeurs ont eu le mérite d'atteindre tous ces objectifs, parmi d'autres en mettant à jour le fait qu'il convenait de privilégier les fonctions carboxyliques des acides aspartique et glutamique de la chaîne collagénique, en tant que sites de greffage de fonctions mercaptoaminées à l'origine des propriétés de réticulation par pontage S-S entre les chaînes de collagène.

Ainsi, la présente invention concerne, tout d'abord, un peptide collagénique modifié par greffage de fonctions thiol libres ou substituées, portées par des restes mercaptoaminés, caractérisé en ce que ces restes mercaptomaminés sont identiques ou différents entre eux et sont exclusivement greffés sur les acides aspartique et glutamique de la chaîne collagénique par l'intermédiaire de liaisons amides, et en ce que ledit peptide collagénique modifié est soluble en milieu aqueux et/ou dans les solvants polaires.
Le fait que les fonctionnalités de réticulation soient portées par les restes carboxyle des acides aspartique et glutamique, confère au peptide collagénique selon l'invention des propriétés avantageuses tout à fait inattendues sur le plan mécanique et biologique. En effet, ce peptide collagénique, modifié peut, dès lors qu'il se trouve sous forme réduite thiol, être réticulé de manière contrôlée, en atteignant des taux de réticulation lui procurant la stabilité ainsi que de bonnes propriétés mécaniques et une biodégradabilité modulable. De plus, les résidus lysine n'étant pas impliqués dans le greffage des résidus mercaptoaminés, ils peuvent servir de sites d'ancrage pour d'autres groupements et procurer au produit des fonctionnalités diverses et variées utiles dans les applications visées.
Dans le cas où le peptide collagénique correspond à du collagène natif avec ou sans télopteptide, le taux de fonctionnalisation par des restes mercaptoaminés peut atteindre 9 à 12 % en nombre, puisque cela correspond au ratio d'acides aminés de type acide aspartique ou acide glutamique constitutifs du collagène. Sont comptabilisés dans ce ratio, les asparagines et les glutamines dont les amides sont susceptibles d'être hydrolysés pour former les dérivés acides correspondants.
Suivant une caractéristique avantageuse de l'invention, ce taux élevé de greffage n'est pas incompatible avec une solubilité importante des formes réticulables (non-réticulées) du collagène modifié, en milieu aqueux et/ou dans les solvants polaires et dans une large gamme de pH. Cela lui confère une grande facilité de mise en oeuvre.

Pour pouvoir réticuler par pontage disulfure les fonctionnalités mercaptoaminées modifiées selon l'invention doivent se trouver sous forme réduite, c'est-à-dire sous forme thiol -(SH). C'est donc lorsqu'ils seront sous cette forme que l'on pourra qualifier les peptides collagéniques modifiés de "réticulables". Ce terme traduit l'aptitude des peptides collagéniques modifiés à s'autoréticuler spontanément en présence de l'oxygène de l'air, à température ambiante et éventuellement en présence d'agents auxiliaires tels que des oxydants.

Les restes mercaptoaminés porteurs des fonctions de réticulation de type thiol libre ou de leurs précurseurs sous forme thiol substitué, sont avantageusement des résidus qui dérivent de près ou de loin de la cystéine ou de ses analogues : cystéamine et homocystéine. Il est intéressant de noter que ces différents résidus mercaptoaminés sont de nature biologique.
Dans le présent exposé, on distingue deux types de restes monovalents mercaptoaminés ou greffons, à savoir ceux porteurs de fonctions thiol directement réticulables et ceux porteurs de fonctions mercapto précurseurs desdites fonctions thiol.

S'agissant des mercaptans précurseurs de thiols, ils déterminent une *première sous famille* de peptides collagéniques modifiés selon l'invention caractérisé en ce qu'au moins une partie des restes mercaptoaminés, greffés sur les acides carboxyliques des acides aspartiques et glufamiques, répond à la formule générale (**I**) suivante : 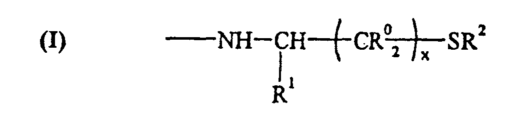 dans laquelle :
- x = 1 ou 2 ;
- R⁰ = H ou CH₃ ;
- R¹ représente H ou COOR³ avec R³ correspondant à un radical hydrocarboné de type aliphatique, aromatique ou alicyclique, de préférence alkylique, alcénylique, arylique, aralkylique, alkylarylique, aralcénylique, alcénylarylique et, plus préférentiellement encore, de type méthylique ou éthylique ;
- R² est un radical aliphatique et/ou alicylique et/ou aromatique, de préférence un alkyle ou un acyle éventuellement soufré et/ou aminé, et plus préférentiellement encore R² répond à la formule **(II)** suivante : 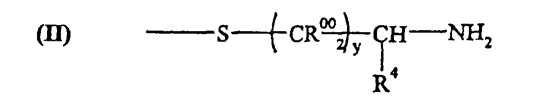 avec y, R⁰⁰ et R⁴ répondant à la même définition que celle donnée en légende dans la formule (I) pour x, R⁰ et R¹.

Plus précisément, les restes mercaptoaminés greffés de ces peptides collagéniques, non immédiatement réticulables, sont choisis dans le groupe des radicaux monovalents comprenant :

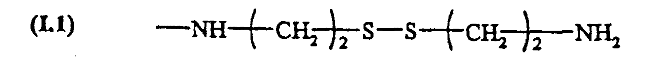

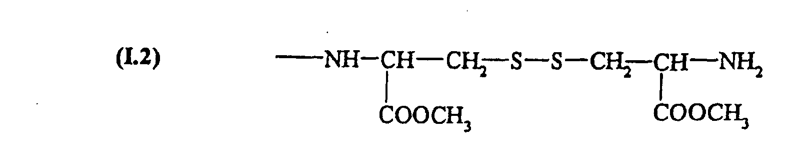

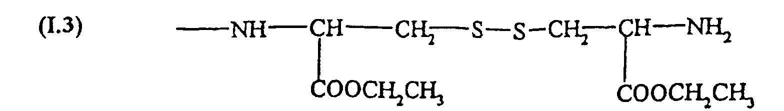

Ce sont des greffons dérivant de la cystine et comprenant donc un pont disulfure réductible à l'aide d'agents réducteurs connus tels que des mercaptans (mercaptoéthanol, acide mercaptoacétique, mercaptoéthylamine, benzylmercaptan, thiorésol, dithiothréitol...) et/ou des sels réducteurs (NaBH₄, Na₂SO₃...) et/ou des réducteurs organiques (phosphine).
Ces nouveaux intermédiaires collagéniques modifiés selon cette *première sous-famille,* sont stables et solubles dans l'eau et plus généralement en milieu aqueux et/ou dans les solvants polaires. En outre, ils sont aisément purifiables et isolables, ce qui en fait des produits pratiques à conditionner, à stocker et à mettre en oeuvre.

La *deuxième sous-famille* de peptides collagéniques modifiés selon l'invention regroupe ceux dont les carboxyles des acides glutamique et aspartique ont réagi avec les fonctions amines des restes mercaptoaminés de formule **(I)** dans lesquels le substituant R² correspond à l'hydrogène.
Les peptides collagéniques modifiés selon la *deuxième sous-famille* peuvent être préparés par réduction des peptides collagéniques selon la *première sous-famille,* à l'aide d'agents réducteurs, tels que ceux définis ci-dessus.
Ces peptides collagéniques réduits sont aisément purifiables et isolables. Dès lors qu'ils sont obtenus sous forme sèche après un isolement en milieu acide, ces peptides sont stables. Enfin, ils sont solubles dans l'eau et plus généralement en milieu aqueux et/ou dans les solvants polaires et ils sont aisés à mettre en oeuvre.
Les reste mercaptoaminés de ces peptides à fonction thiol libres sont définis par la formule (I') suivante : 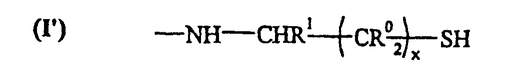 dans laquelle R¹ peut correspondre à H ou COOR³, avec x, R¹, R⁰, R³ tels que définis supra et, en outre, R³ peut représenter l'hydrogène ou un cation pour former un sel avec COO⁻, ce cation étant, de préférence, Na⁺, K⁺, Li⁺, dans le cas où l'on prévoit une étape de déprotection de l'ester. Le greffon ainsi mis en oeuvre dérive directement de la cystéine.
Les peptides collagéniques comprenant de tels restes mercaptoaminés à fonctions réactives thiol ont pour caractéristique d'être réticulables au sens de l'invention.
La réticulation s'opère par oxydation des thiols en ponts disulfures, ce qui permet d'obtenir un réseau collagénique tridimensionnel réticulé chimiquement, insoluble dans les milieux physiologiques et parfaitement stable. Cette oxydation peut être obtenue e.g. par l'oxygène de l'air en milieu faiblement basique, par l'eau oxygénée, ou par des dérivés iodés (iode, bétadine).

Parmi les peptides collagéniques modifiés conformes à l'invention, on peut isoler ceux qui existent sous forme réticulée et qui composent une *troisième sous famille* de peptides collagéniques comprenant des chaînes collagéniques reliées entre elles par des ponts disulfure, dont les atomes de soufre constitutifs appartiennent à des restes mercaptoaminés greffés sur les acides aspartique et glutamique des chaînes collagéniques, exclusivement par l'intermédiaire de liaisons amides.
Ces peptides collagéniques réticulés de la *troisième sous-famille* peuvent être obtenus, avantageusement, à partir des peptides collagéniques modifiés de la *deuxième sous-famille.*

Ces peptides collagéniques réticulés sont des produits nouveaux, stables et dont les qualités mécaniques et biologiques en font des biomatériaux de choix pour la réalisation d'articles médicaux ou chirurgicaux tels que des implants, des prothèses, des pansements ou bien encore de la peau artificielle. Dans la mesure où il est possible de jouer sur le taux de substitution des motifs carboxyliques des acides aspartiques et glutamiques, on dispose d'une certaine marge de manoeuvre pour choisir la qualité mécanique et vitesse de biodégradation appropriées.

Par ailleurs, l'état réticulé dont il est question pour ces peptides collagéniques appartenant à la *troisième sous famille* décrite dans le présent exposé, est réversible. En effet, il est possible de réduire les ponts disulfure à l'aide d'agents réducteurs appropriés. Des exemples de ces réducteurs sont donnés ci-dessus.

Conformément à l'invention les restes carboxyliques libres des monomères acides aspartique et glutamique de la chaîne collagénique sont mobilisés pour le greffage de fonctionnalités de réticulation. Mais il n'en reste pas moins qu'au moins une partie des autres fonctions libres de la chaîne collagénique, comme par exemple les fonctions amine des résidus lysine, peuvent servir de sites d'ancrage, pour des groupements qui différent des restes mercaptoaminés tels que définis ci-dessus et qui procurent des fonctionnalités diverses et variées, utiles dans les applications visées. D'où il s'ensuit que les peptides collagéniques tels que définis supra peuvent comporter, selon une variante, des greffons G fixés sur au moins une partie des motifs amine libres de la chaîne collagénique, par l'intermédiaire de liaisons amides, G étant un acyle comprenant une entité hydrocarbonée, A L'EXCLUSION des restes mercaptoaminés, notamment ceux tels que définis supra, cette entité comprenant, éventuellement, des hétéroatomes (avantageusement O et/ou N) et étant, de préférence, choisie parmi les alkyles et/ou les alcényles et/ou les alicycliques et/ou les aromatiques et, plus préférentiellement encore, parmi les groupements comprenant une chaîne alkyle, éventuellement insaturée, comprenant de 1 à 22 carbone(s) ou répondant à la formule **(III)** suivante : 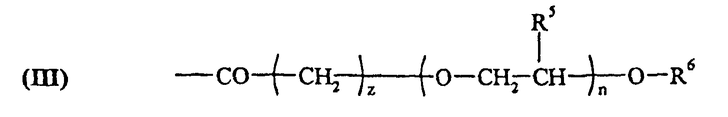 avec :
- R⁵ = H ou CH₃ ;
- R⁶ = H, un radical alkyle linéaire ou ramifié et de préférence un méthyle ;
- z = 0,1 ou 2 et n > 0.

Le nombre n d'unités récurrentes est choisi de telle sorte que le poids moléculaire de la chaîne polymère soit compris entre 100 et 15 000, de préférence entre 200 et 8 000 et, par exemple, soit de l'ordre de 4 000.

Cette fonctionnalisation supplémentaire sur les sites amine des lysines peut conférer au peptide collagénique modifié un caractère hydrophile ou hydrophobe, voire tensioactif, ce qui permet de moduler les propriétés de gonflement, de résistance mécanique et de cinétique de dégradation. Il est également concevable que cette fonctionnalisation ait des visées thérapeutiques grâce à l'ancrage d'un principe actif.

Outre l'aspect produit collagénique pris en tant que tel, la présente invention a également trait à l'obtention de peptides collagéniques modifiés et, notamment, ceux tels que définis ci-dessus et, plus particulièrement encore, ceux appartenant aux *trois sous familles* présentées ci-avant.

L'invention concerne ainsi un procédé d'obtention d'un peptide collagénique modifié par greffage de fonctions thiols, libres ou substituées et portées par des restes mercaptoaminés. Ce procédé consiste essentiellement à faire réagir en solution le peptide collagénique avec au moins un précurseur d'un reste mercaptoaminé dont la fonction thiol et l'éventuelle fonction carboxylique sont bloquées, en présence d'au moins un agent de greffage, de préférence choisi dans le groupe des produits activant les groupements carboxyliques et, plus préférentiellement encore, parmi les carbodiimides.

Les conditions d'obtention sont choisies de telle sorte que le greffage du reste mercaptoaminé s'opère sur les motifs carboxyliques libres des acides aspartiques et glutamiques de la chaîne collagénique.

Ce procédé est particulièrement original et avantageux en ce qu'il peut être réalisé en milieu aqueux dans lequel sont au moins partiellement solubilisés les produits de départ et/ou les produits intermédiaires et/ou les collagènes modifiés finaux.
En pratique, tous les produits contenus dans le milieu réactionnel aqueux sont solubilisés dans celui-ci, de la première à la dernière étape.
Cette synthèse en milieu aqueux, conformément à l'invention, est particulièrement intéressante sur le plan industriel, car elle est très simple à mettre en oeuvre, peu coûteuse et non polluante. En effet, il est aisé, par exemple, d'éliminer les réactifs (e. g. par diafiltration) et de récupérer le collagène modifié visé.

Le procédé selon l'invention est d'autant plus intéressant qu'il permet d'atteindre de forts taux de greffage de restes mercaptoaminés (e. g. 12 %). De préférence, les restes mercaptoaminés (groupements monovalents) qui sont greffés sur le peptide collagénique, sont ceux tels que définis supra, notamment par les formules **(I), (I.1), (I.2)** et **(I.3).**

En pratique, les peptides collagéniques ainsi obtenus correspondent, e. g. aux précurseurs, tels que visés ci-dessus, de peptides collagéniques réticulables.
Ces précurseurs sont compris dans la *première sous-famille* de peptides collagéniques modifiés selon l'invention.

Pour pouvoir réagir avec les motifs carboxyliques libres du peptide collagénique, le greffon mercaptoaminé possède une fonction amine libre apte à réagir avec les COOH pour former une liaison amide. Ce précurseur est, par exemple, une cystéine, une homocystéine ou une cystéamine dont la fonction thiol et l'éventuelle fonction acide carboxylique est (sont) correctement protégée(s). Un moyen efficace de protection de la fonction thiol est de choisir comme précurseur du reste mercaptoaminé à greffer, la cystine, l'homocystine ou la cystamine, qui comprennent un pont disulfure stabilisateur de la fonction mercapto. Comme autre moyen de protection de cette dernière, on peut choisir toute fonction classique de protection des thiols connue dans l'art antérieur (voir, par exemple, "Greene : *Protecting Groups in Organic Chemistry*, WILEY, 1975").

Les éventuelles fonctions COOH du greffon peuvent quant à elles être protégées à l'aide d'un groupement protecteur ou toute autre fonction organique pouvant apporter une propriété quelconque intéressante (les PEG, groupements hydrophobes ou hydrophiles ou chargés).

Selon une disposition avantageuse de l'invention, le précurseur du reste mercaptoaminé répond à une formule **(IV)** correspondant à la formule **(I)** donnée supra et dans laquelle la valence libre est remplacée par un substituant apte à réagir avec les fonctions carboxyliques des acides aspartique et glutamique de la chaîne collagénique, ce substituant étant de préférence l'hydrogène, de façon à ce que la fonction réactive soit une amine primaire. Les précurseurs de formule **(IV)** tout spécialement préférés sont la cystamine **(I.1),** la cystine-diméthylester **(I.2)** et la cystine diéthylester (**I.3**), qui comprennent toutes trois un pont disulfure protégeant la fonction thiol.

En pratique, le greffage du reste mercaptoaminé s'effectue en procédant à la dissolution du peptide collagénique, puis du précurseur du reste mercaptoaminé à greffer dans un solvant approprié. Il peut s'agir, par exemple, de l'eau (de préférence) et/ou d'un solvant organique, comme le DiMéthylSulfOxyde (DMSO), la N-MéthylPyrrolidone (NMP) ou autres.

Les conditions réactionnelles sont choisies afin d'éviter que le collagène activé ne réagisse avec les amines contenues dans son propre squelette.
La solution réactionnelle est ensuite additionnée d'un agent de couplage, tel qu'un carbodiimide, et on laisse le greffage s'opérer en maintenant le milieu sous agitation pendant quelques heures, à température ambiante.

Le procédé selon l'invention permet d'obtenir des peptides collagéniques substitués par des restes mercaptoaminés précurseurs des restes thiols réticulables. Les peptides ainsi obtenus sont de nouveaux produits intermédiaires stables et solubles dans l'eau. Us peuvent être isolés et purifiés, par exemple par dialyse, diafiltration puis lyophilisation ou par précipitation en milieu organique puis par séchage.

La présente invention a également pour objet un procédé de préparation d'un peptide collagénique réticulable modifié par greffage de fonctions thiol libres portées par des restes mercaptoaminés. Ce procédé est caractérisé en ce qu'il consiste, essentiellement :
**1.** à faire réagir en solution le peptide collagénique avec au moins un précurseur d'un reste mercaptoaminé dont la fonction thiol et l'éventuelle fonction carboxylique sont bloquées, en présence d'au moins un agent de greffage, de préférence choisi dans le groupe comprenant les produits activant les groupements carboxyliques, de préférence les carbodiimides,
**2.** et à déprotéger (transformation en thiols) les fonctions mercapto des restes mercaptoaminés greffés sur les peptides collagéniques modifiés obtenus à l'étape **1**.
Les peptides collagéniques réticulables ainsi préparés correspondent, par exemple, aux produits à fonctions thiol libres compris dans la *deuxième sous-famille* de peptides collagéniques modifiés, tels que définis supra.

Dans le cas où la protection ou le masquage des fonctions mercapto est assuré par un pont disulfure (c'est-à-dire quand les précurseurs des greffons sont e.g. la cystamine ou la cystine) la régénération de la fonction thiol se fait par réduction. Cette dernière peut être réalisée à l'aide d'agents réducteurs tels que des mercaptans (mercaptoéthanol, acide mercaptoacétique, mercaptoéthylamine, benzylmercaptan, thiolcrésol, dithiothréitol, ....) et/ou des sels réducteurs (NaBH₄, Na₂SO₃.....) et/ou des réducteurs organiques (phosphine).

Suivant une caractéristique préférée de la présente invention, on procède à une réduction du pont disulfure de protection en milieu aqueux basique à l'aide de dithiothréitol. Après cette étape, le collagène thiolé obtenu est purifié par dialyse/diafiltration et peut être isolé e.g. par lyophilisation.

L'invention concerne, également, un procédé de préparation d'un peptide collagénique réticulé, à partir d'un peptide collagénique modifié par greffage de fonctions thiol libres et portées par des restes mercaptoaminés. Ce procédé est caractérisé en ce qu'il consiste, essentiellement :
**1.** à faire réagir en solution le peptide collagénique avec au moins un précurseur d'un reste mercaptoaminé dont la fonction thiol et l'éventuelle fonction carboxylique sont bloquées, en présence d'au moins un agent de greffage, de préférence choisi dans le groupe comprenant les produits activant les groupements carboxyliques, de préférence les carbodiimides,
**2.** et à déprotéger (transformation en thiols) les fonctions mercapto des restes mercaptoaminés greffés sur les peptides collagéniques modifiés obtenus à l'étape **1**,
**3.** et à oxyder les fonctions thiol du peptide collagénique modifié réticulable issu de l'étape **2**., de façon à former des ponts disulfure intercaténaires.
Cette oxydation peut être e. g. effectuée à l'aide de l'oxygène de l'air en milieu faiblement basique, ou à l'aide d'eau oxygénée ou de dérivés iodés (iode, bétadine).
Les peptides collagéniques réticulés, tels que préparés par le procédé ci-dessus, correspondent notamment aux produits réticulés de la *troisième sous-famille* de peptides collagéniques modifiés, tels que définis ci-avant.

Suivant une caractéristique avantageuse propre aux trois procédés décrits ci-dessus, on prévoit une étape complémentaire F de fonctionnalisation par des greffons G de nature différente de celle des greffons fixés aux fonctions carboxyliques des acides aspartiques et glutamiques, cette étape F consistant essentiellement à procéder à une acylation d'au moins une partie des fonctions amines libres de la chaîne collagénique, de façon à fixer sur celles-ci des greffons G comprenant une entité hydrocarbonée, A L'EXCLUSION des restes mercaptoaminés, notamment ceux tels que définis supra, cette entité comprenant éventuellement des hétéroatomes (avantageusement O et/ou N) et étant, de préférence, chcisie parmi les alkyles et/ou les alcényles et/ou les alicycliques et/ou les aromatiques et, plus préférentiellement encore, parmi les groupements comprenant une chaîne alkyle, éventuellement insaturée ou répondant à la formule (**III**) suivante : 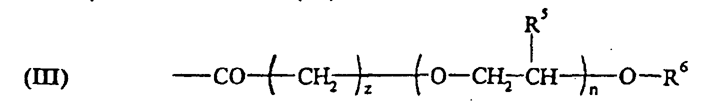 avec :
- R⁵ = H ou CH₃ ;
- R⁶ = H, un radical alkyle linéaire ou ramifié et de préférence un méthyle ;
- z = 0,1 ou 2 et n > 0.

Pour pouvoir réagir par acylation avec les fonctions amine libres du résidu de la chaîne collagénique, les précurseurs des greffons G comportent, avantageusement, au moins une fonction acide carboxylique activable.

Il est préférable que cette acylation se déroule avant ia réaction des fonctions carboxyliques libres de la chaîne collagénique avec le précurseur du greffon mercaptoaminé **(I)**. Ceci étant précisé, il n'est pas à exclure que cette acylation intervienne également sur les peptides collagéniques thiolés issus de l'étape **1** et/ou sur les peptides collagéniques réticulés issus de l'étape **3**. (e. g. directement sur un film réticulé, avec élimination des réactifs par simple lavage).

Les réactions d'acylation et de couplage de fonctions amine avec des sites carboxyliques appartenant à des protéines, sont connues de l'homme du métier dans le domaine de la biochimie des protéines. Pour plus de détails à cet égard, on se référera notamment aux ouvrages suivants :
- "Techniques in protein modification" R.L LUNDBLAD, Boca Raton, CRC Press, 1995, Chap 10-14.
- *"Chemistry of protein conjugation and cross-linking"* S. S. WONG, Boca raton, CRC Press, 1993, Chap. 2.

Il est intéressant de noter que les réactifs utilisés pour les modifications chimiques opérées selon les procédés conformes à l'invention, sont soit transformables en produits non toxiques, soit facilement éliminables par des procédés non dégradants comme par exemple la dialyse.

Par ailleurs, l'invention offre la possibilité très appréciable de contrôle de la cinétique et du taux de réticulation du collagène.

Un autre avantage non négligeable de la présente invention est qu'elle permet de moduler les propriétés mécaniques et la biodégradation en contrôlant le nombre des résidus mercaptoaminés introduits par unité de masse du collagène.

Il est également intéressant de pouvoir fonctionnaliser les chaînes collagéniques réticulées ou non à l'aide de fonctions hydrophiles, par exemple.

Enfin, il est important de signaler que les produits selon l'invention sont stérilisables par les méthodes classiques de stérilisation de polymères biologiques.

On insistera pour terminer sur la très bonne solubilité en milieu aqueux des nouveaux peptides collagéniques non réticulés selon l'invention, qui ont pour caractéristique de posséder des fonctions de réticulation soufrées portées exclusivement par les carboxyles des acides aspartiques et des glutamiques.

Il s'ensuit que les produits réticulables selon l'invention trouvent des applications immédiates, d'une part, en médecine humaine et, d'autre part, dans le domaine de la biologie.

En médecine humaine, il peut s'agir d'implants, par exemple ophtalmologiques, de prothèses, (par exemple osseuses), de pansements sous forme de films ou de feutres, de tissus artificiels (épiderme, vaisseaux, ligaments, os), de systèmes de bioencapsulation (microsphères, microcapsules) permettant la libération contrôlée de principes actifs in vivo, de revêtements de biocompatibilisation d'articles médicaux implantables ou bien, encore, de fils de suture. Les produits collagéniques réticulables selon l'invention peuvent également être utilisés en chirurgie, comme colles et/ou comme matériaux d'étanchéification (ciments).

En biologie, les matériaux suivant l'invention constituent d'excellents supports pour cultures cellulaires bidimensionnelles (films) et tridimensionnelles (feutres).

Le collagène réticulé selon l'invention peut être utilisé seul ou en mélange, par exemple, avec des polymères biologiques modifiés ou non ou des polymères synthétiques.

Pour chacune des applications biomédicales précitées, il est indispensable de disposer d'un collagène, réticulé, possédant des propriétés physicochimiques, mécaniques ou biologiques déterminées et spécifiques. Par conséquent, il est nécessaire de maîtriser parfaitement les modifications chimiques du collagène, de manière à pouvoir produire une large gamme de collagènes réticulables et répondre ainsi, de façon satisfaisante, à la plupart des contraintes apparaissant lors de l'élaboration du cahier des charges d'une application donnée. Il ressort de la description ci-dessus que l'invention répond parfaitement à cette nécessité.

D'autres avantages et variantes de la présente invention ressortent bien des exemples de mise en oeuvre donnés ci-après.

### EXEMPLES

### EXEMPLE 1 : SYNTHESE D'UN PEPTIDE COLLAGENIQUE (2^{ème} SOUS-FAMILLE) DONT LES ACIDES CARBOXYLIQUES SONT SUBSTITUES PAR LA CYSTEINE-ETHYLESTER (TAUX DE SUBSTITUTION REPRESENTANT 0,8 % MOLAIRE DES ACIDES AMINES).

### 1) Etape I : couplage (obtention I^{ère} sous-famille):

25 g d'atélocollagène (types I + III, extrait de peaux de veaux, 1,3 mmol de COOH/g) sont placés dans 2,5 l d'eau et la température du milieu est amené à 50 °C sous agitation. La solution à 1 % p/v ainsi obtenue est filtrée sur 0,22 µm.
Une fois la température abaissée à 30 °C, 46,5 g de cystine-diéthylester sont ajoutés et le pH est ajusté à 4,2. On ajoute alors 0,6 g de 1-(3-diméthylaminopropyl)-3-éthylcarbodiimide HCl et on laisse la réaction se dérouler pendant 2 h à 30 °C sous agitation. Le milieu réactionnel est concentré à 5 % p/v et dialysé contre de l'eau pour éliminer l'excès de réactifs et les sous-produits de la réaction. Le produit obtenu est un intermédiaire stable de synthèse. Il s'agit d'un peptide collagénique (1^{ère} sous-famille) dont une fraction des acides aspartiques et glutamiques sont substitués par de la cystine-diéthylester.
Le taux de substitution est mesuré par un dosage par le NSTB (2-nitro-5-thiosulfobenzoate), réactif des ponts disulfure. Ce dosage est décrit dans : Thannhauser T. W. et al., Analysis of disulfide bonds in peptides and proteins. *Methods in Enzymology*. Jacoby W. B. and Griffith O. XL New-York : Academic Press, 1987. Vol. 143, 115-119.
[S-S] : 0,094 mmol/g de produit sec ; soit 0,87 % molaire d'acides aminés substitués. Le produit obtenu peut être isolé par lyophilisation ou être réduit pour conduire au collagène thiol correspondant.

### 2) Etape II : réduction (obtention 2^{ème} sous-famille) :

Au peptide collagénique modifié en solution à 5 % p/v dans l'eau obtenu à l'étape I, on ajoute 7,6 g de glycine, 5,8 g de 1,4-dithiothréitol et qsp NaOH 4 N pour atteindre un pH de 9,0. Le milieu réactionnel est maintenu trois heures sous agitation à 35 °C. A ce stade, la solution est acidifiée à pH 2 par HCl 6 N, dialysée contre HCl 0,012 N pour éliminer toute trace de réactifs et de sous-produits de réaction, puis filtrée sur 0,22 µm. Le produit ainsi purifié est isolé par lyophilisation.
Le taux de substitution est mesuré par un dosage par l'acide 5,5'-dithiobis-2-nitrobenzoïque (DTNB), réactif spécifique des fonctions thiol. Ce dosage est décrit dans : "Ellman G. L., Tissue sulfhydryl groups, *Archives of Biochemistry and Biophysics,,* 1959, 82, 70-77".
[SH] : 0,091 mmol / g de produit sec, soit 0,8 % molaire d'acides aminés substitués. Toute la synthèse peut être réalisée aseptiquement de manière à obtenir *in fine* le produit sous forme d'un lyophilisat stérile.

### EXEMPLE 2 : SYNTHESE D'UN PEPTIDE COLLAGENIQUE (2^{ème} SOUS-FAMILLE) DONT LES ACIDES CARBOXYLIQUES SONT SUBSTITUES PAR LA CYSTEINE-ETHYLESTER (TAUX DE SUBSTITUTION REPRESENTANT 3 % MOLAIRE DES ACIDES AMINES).

On reproduit l'exemple 1, à la seule exception que la quantité d'agent de couplage est de 2,9 g.
[SH] : 0.347 mmol / g de produit sec, soit 3.3 % molaire d'acides aminés substitués.

### EXEMPLE 3 : SYNTHESE D'UN PEPTIDE COLLAGENIQUE (2^{ème} SOUS-FAMILLE) DONT LES ACIDES CARBOXYLIQUES SONT SUBSTITUES PAR LA CYSTEINE-ETHYLESTER (TAUX DE SUBSTITUTION REPRESENTANT 7 % MOLAIRE DES ACIDES AMINES).

On reproduit l'exemple 1, à la seule exception que la quantité d'agent de couplage est de 12 g.
[SH] : 0,706 mmol / g de produit sec, soit 7 % molaire d'acides aminés substitués.

### EXEMPLE 4 : SYNTHESE D'UNE GELATINE (2^{ème} SOUS-FAMILLE) DONT LES ACIDES CARBOXYLIQUES SONT SUBSTITES PAR LA CYSTEINE-ETHYLESTER (TAUX DE SUBSTITUTION REPRESENTANT 5 % MOLAIRE DES ACIDES AMINES).

On reproduit l'exemple 1, en remplaçant l'atélocollagène par de la gélatine (gélatine extraite de peaux de porcs, 250 bloom, 1 mmol de COOH/g).
[SH] : 0,536 mmol/g de produit sec, soit 5,2 % molaire d'acides aminés substitués.

### EXEMPLE 5 : SYNTHESE D'UN PEPTIDE COLLAGENIQUE (2^{ème} SOUS-FAMILLE) DONT LES ACIDES CARBOXYLIQUES SONT SUBSTITUES PAR LA CYSTEAMINE (TAUX DE SUBSTITUTION REPRESENTANT 3 % MOLAIRE DES ACIDES AMINES).

On reproduit l'exemple 1, en remplaçant 46,5 g de cystine-diéthylester par 28,4 g de cystamine.
[SH] : 0,33 mmol/g de produit sec, soit 3,0 % molaire d'acides aminés substitués.

### EXEMPLE 6 : SYNTHESE D'UN PEPTIDE COLLAGENIQUE (2^{ème} SOUS-FAMILLE) DONT LES AMINES SONT ACETYLEES (GREFFON G) ET DONT LES ACIDES CARBOXYLIQUES SONT SUBSTITUES PAR LA CYSTEINE-ETHYLESTER (TAUX DE SUBSTITUTION REPRESENTANT 5 % MOLAIRE DES ACIDES AMINES).

25 g d'atélocollagène (types I + III, extrait de peaux de veaux, 1,0 mmol de COOH/g ; 0,33 mol de ε-NH₂/g) sont placés dans 0,5 l d'eau et la température du milieu est amenée à 50 °C sous agitation. La solution à 5 % p/v ainsi obtenue est filtrée sur 0,22 µm.
Après refroidissement de la solution à 30 °C, on dissous 4,2 g de NaHCO₃ et qsp NaOH 4 N pour ajuster le pH à 8,5. On ajoute alors lentement et séquentiellement, pendant 30 minutes, 7,34 ml d'anhydride acétique, sous agitation à 30 °C, tout en maintenant le pH à 8,5 à l'aide de soude 4 N. On acidifie alors lentement la solution à pH 3 par de l'HCl 6 N et on dialyse contre de l'eau pour éliminer les réactifs en excès. On dilue enfin le milieu à 1 % p/v par de l'eau et on continue la synthèse tel qu'indiqué dans l'exemple 1 (couplage de la cystine-diéthylester puis réduction).
[acétyl] par dosage d'acide acétique (kit Boehringher) après hydrolyse basique du produit : 0,30 mmol/g de produit sec, soit 2,9 % molaire d'acide aminés acétylés (acétylation presque totale des résidus lysine).
[SH] : 0,53 mmol/g de produit, soit 5,1 % molaire d'acides aminés substitués.

### EXEMPLE 7 : SYNTHESE D'UN PEPTIDE COLLAGENIQUE (2^{ème} SOUS-FAMILLE) DONT LES AMINES SONT SUBSTITUEES PAR DU PEG (GREFFON G) ET DONT LES ACIDES CARBOXYLIQUES SONT SUBSTITUES PAR LA CYSTEINE-ETHYLESTER (TAUX DE SUBSTITUTION REPRESENTANT 5 % MOLAIRE DES ACIDES AMINES).

10 g d'atélocollagène (types I + III, extrait de peaux de veaux, 1,.3 mmol de COOH/g ; 0,33 mol de ε-NH₂/g) sont placés dans 0,5 l d'eau et la température du milieu est amené à 50 °C sous agitation. La solution à 2 % p/v ainsi obtenue est filtrée sur 0,22 µm.
Une fois la température abaissée à 30 °C, le pH est ajusté à 9,0 par NaOH 4 N. On ajoute alors 5 g de N-hydroxysuccinimidylester du méthoxypolyéthylèneglycol acide propionique (SPA-PEG) de PM 5 000 g/mol et on laisse la réaction se dérouler à 30 °C sous agitation pendant 30 min, tout en maintenant le pH à 9 par un ajout de NaOH 4 N. On ajoute à nouveau 5 g de SPA-PEG et on agite le milieu réactionnel pendant 30 min en maintenant le pH. Le milieu est ensuite dilué au ½ par de l'eau pour ramener la concentration du collagène à 1 % p/v.
18,5 g de cystine-diéthylester sont ajoutés et le pH est ajusté à 4,2. On ajoute alors 2,2 g de 1-(3-diméthylaminopropyl)-3-éthylcarbodiimide. HCl et on laisse la réaction se dérouler pendant 2 h à 30 °C sous agitation. Le milieu réactionnel est concentré à 5 % p/v et dialysé contre de l'eau pour éliminer l'excès de réactifs et les sous-produits de la réaction.
Au peptide collagénique modifié en solution à 5 % p/v dans l'eau, on ajoute 3,0 g de glycine, 2,3 g de 1,4-dithiothréitol et qsp NaOH 4 N pour atteindre un pH de 9,0. Le milieu réactionnel est maintenu trois heures sous agitation à 35 °C. A ce stade, la solution est acidifiée à pH 2 par HCl 6 N, dialysée contre HCl 0,012 N pour éliminer toute trace de réactifs et de sous-produits de réaction puis filtrée sur 0,22 µm. Le produit ainsi purifié est isolé par lyophilisation.
Le lyophylisat est extrait par 2 l d'éthanol absolu, contracté par de l'acétone puis séché sous vide à 30 °C pendant 18 h.
L'absence de polyethylèneglycol non greffé est contrôlée par chromatographie de perméation sur gel, détection réfratométrique.
[SH] : 0,247 mmol/g de produit sec, soit 4,5 % molaire de substitution des acide aminés.
[PEG] : substitution de 0,8 % molaire des. acides aminés, taux recalculé d'après la quantité d'OH-proline dosée dans le produit modifié / produit non modifié.

### EXEMPLE 8 : SOLUBILITE DES PEPTIDES COLLAGENIQUES MODIFIES.

250 mg du peptide collagénique sont placés dans 5 g d'eau ppi, et on agite en flacon fermé pendant 15 min à 40 °C. Les mesures de pH sont réalisée, à 30 °C. Les ajustements de pH sont réalisés à l'aide de NaOH 1N. Quelques exemples de solubilité sont présentés dans le Tableau 1.

**TABLEAU 1 :**

| **PEPTIDE COLLACENIQUE OBTENU** | **ASPECT INITIAL** | **SOLUBILITE** |
|---|---|---|
| **Exemple 1** | pH 2,1 solution limpide | pas de zone d'insolubilité pour un pH allant de 2,5 à 10 |
| **Exemple 3** | pH 2,2 solution limpide | pas de zone d'insolubilité pour un pH allant de 2,5 à 10 |
| **Exemple 5** | pH 1,9 solution limpide | pas de zone d'insolubilité pour un pH allant de 2,5 à 10 |
| **Exemple 7** | pH 2,5 gel transparent | fluidification au fur et à mesure que l'on augmente le pH. Solution fluide à partir de pH 6 |

### EXEMPLE 9 : RETICULATION DES PEPTIDES COLLAGENIQUES (2^{ème} SOUS-FAMILLE) PAR OXYDATION : FORMATION DE GELS (3^{ème} SOUS-FAMILLE).

Le procédé utilisé est le même quel que soit le peptide collagénique utilisé (exemples 1, 3, 7).
250 mg de lyophilisat sont placés dans 4,5 ml de tampon phosphate salin 10 mM pH 7,4 (PBS) et le mélange est agité en flacon fermé à 40°C pendant 15 minutes. Le pH est ajusté à 7,4 ± 0,1 à l'aide de NaOH 1 N, et la quantité de PBS nécessaire pour obtenir une concentration finale en peptide collagénique de 50 g/l est ajouté. Les échantillons sont placés à 37 °C. A 900 µl de la solution de peptide collagénique sont ajoutés 100 µl d'une solution de H₂O₂ l % dans du PBS préchauffé à 37 °G. Les indications du Tableau 2 montrent que la réticulation par oxydation (cinétique et taux), dans des conditions données, dépend du peptide collagénique modifié utilisé.

**TABLEAU 2 :**

| **PEPTIDE COLLAGENIQUE OBTENU** | **TEMPS DE PRISE DU GEL (37 °C)** | **DESCRIPTION DU GEL (37 °C)** |
|---|---|---|
| **Exemple 1** | 20 secondes | gel homogène transparent souple |
| **Exemple 3** | 5-10 secondes | gel homogène turbide |
| **Exemple 7** | 1 minutes 15 secondes | gel homogène transparent mou et collant |

### EXEMPLE 10 : RETICULATION DES PEPTIDES COLLAGENIQUES PAR OXYDATION : FORMATION DE FILMS.

Le procédé de préparation du film est identique quelque soit le peptide collagénique utilisé.

### Etape 1 :

Une solution à 20g/l de peptide collagènique précurseur, est préparée par dissolution du lyophilisat dans de l'eau stérile. Dans cet exemple 2,0 g de lyophilisat sont dissous dans 98 g d'eau stérile. Le mélange est agité dans un récipient fermé à 40°C pendant 15 min, afin d'obtenir une dissolution complète. Le pH de la solution est ajusté à 6,5 avec de la soude 1N, à 25 °C. La solution est remise en agitation à 40 °C pendant 10 min.

### Etape 2 :

La solution à 40 °C est filtrée sur membranes de porosité 0,45 µm, puis sur membranes de porosité 0,2 µm. La dernière filtration s'effectue au dessus des moules stériles (on peut utiliser des boites de Pétri en polystyrène).

### Etape 3 :

40,0 g de solution filtrée sont coulés dans deux moules de 12 × 12 cm². Les moules sont refermés.

### Etape 4 :

On réalise la maturation de la solution, qui se traduit par une gélification physique, pendant 24 h à une température de 16 °C ± 1. Cette température est nécessairement inférieure à la température de transition gel/sol. La maturation est effectuée dans une enceinte à température régulée, les moules reposent sur une plaque horizontale.

### Etape 5 :

Après 24 h, les couvercles des moules sont retirés et l'évaporation des solutions gélifiées s'effectue sur 24 h, à la même température dans une enceinte confinée, en présence d'agents dessicants (typiquement des pastilles de soude). Au bout de 24 h, les films obtenus sont secs, limpides et lisses.

### Etape 6 :

La réticulation des films secs est effectuée à 20 °C, en versant 30 g de solution de peroxyde d'hydrogène à 0,3 % dans une solution aqueuse décimolaire d'acétate d'ammonium.

### Etape 7 :

Le film réticulé est retiré et lavé successivement par 30 g de tampon phosphate pH 7,4 et 30 g d'eau.
Toutes les solutions utilisées sont stériles.

### Etape 8 :

Le film est ensuite laissé à sécher sous hotte à flux laminaire pendant 24 h. Le film séché obtenu contient un pourcentage d'eau résiduelle de 10 % environ.

Les films obtenus sont stables à température ambiante. Ils restent stables et manipulables après 24 h dans l'eau ou dans un tampon phosphate.

### EXEMPLE 11 : PROPRIETES MECANIQUES EN TRACTION DES FILMS OBTENUS SELON L'EXEMPLE 10.

Les mesures de propriétés mécaniques de films sont effectuées à l'aide d'un appareil d'essais universel de type DY34 de la marque Adamel Lhomargy. Les films sont hydratés à température ambiante dans un tampon phosphate salin (PBS, pH = 7,4) pendant 2 h. Puis ils sont découpés en bandes de 4 mm sur 30 rnm à l'aide d'un emporte pièce très coupant. L'épaisseur est mesurée sur les échantillons hydratés.
Les échantillons sont fixés sur un cadre carton qui aide à la mise en place dans les mâchoires. L'échantillon de film est maintenu hydraté. Le cadre est coupé juste avant l'essai de traction, qui se déroule à vitesse constante de 2 mm/min.
Le module à l'origine ainsi que la contrainte à la rupture sont calculés d'après les courbes de traction en utilisant les sections des éprouvettes hydratées.
Les propriétés en traction des films obtenus selon le procédé décrit à l'exemple 10 dépendent du peptide collagénique modifié utilisé, comme le montre le Tableau 3.

**TABLEAU 3 :**

| **PEPTIDE COLLAGENIQUE OBTENU** | **EPAISSEUR SECHE** (µM) | **EPAISSEUR HUMIDE** (µM) | **F**_{**MAX**} (N) | **ALLONGEMENT** (%) | σ **max** (Mpa) | **MODULE INITIAL** (MPa) |
|---|---|---|---|---|---|---|
| **Exemple 1** | 45 | 153 | 2,9 | 43 | 3,2 | 4,6 |
| **Exemple 2** | 45 | 94,5 | 3,1 | 28,5 | 8,1 | 21,6 |
| **Exemple 3** | 45 | 80 | 5,4 | 42,5 | 16,7 | 25,8 |
| LEGENDE : Fmax = force maximale à la rupture σ max = contrainte maximale à la rupture | | | | | | |

## Revendications

1. Peptide collagénique modifié par greffage de fonctions thiol libres ou substituées portées par des restes mercaptoaminés, caractérisé :
• en ce que ces restes mercaptoaminés sont identiques ou différents entre eux et sont exclusivement greffés sur les acides aspartique et glutamique de la chaîne collagénique par l'intermédiaire de liaisons amide,
• et en ce qu'il est soluble en milieu aqueux et/ou dans les solvants polaires.

2. Peptide collagénique selon la revendication 1, **caractérisé en ce qu'**au moins une partie des restes mercaptoaminés, greffés sur les acides carboxyliques des acides aspartique et glutamique, répond à la formule générale **(I)** suivante : 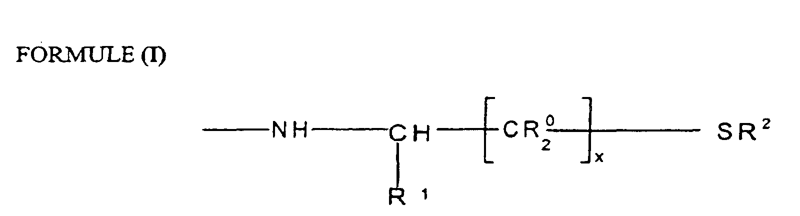 dans laquelle :
• x = 1 ou 2;
• R° = H ou CH₃;
• R¹ représente H ou COOR³ avec R³ correspondant à un radical hydrocarboné de type aliphatique, aromatique ou alicyclique, de préférence alkylique, alcénylique, arylique, aralkylique, alkylaryque, alcénylarylique; et plus préférentiellement encore de type méthylique ou éthylique;
• R² est un radical aliphatique et/ou alicyclique et/ou aromatique, de préférence un alkyle ou un acyle éventuellement soufré et/ou aminé, et plus préférentiellement encore R² répond à la formule **(II)** suivante: 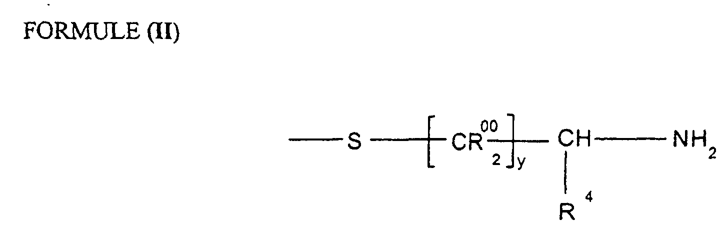
• avec y, R⁰⁰ et R⁴ répondant à la même définition que celle donnée en légende dans la formule (I) pour x, R⁰ et R¹.

3. Peptide collagénique selon la revendication 2 **caractérisé en ce que** les restes mercaptoaminés greffés sont choisis dans le groupe des radicaux suivants :

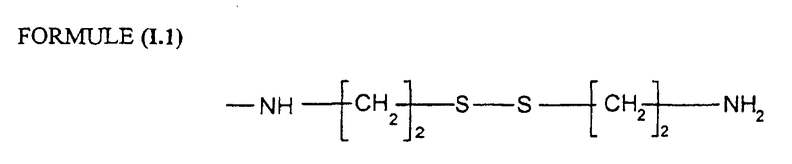

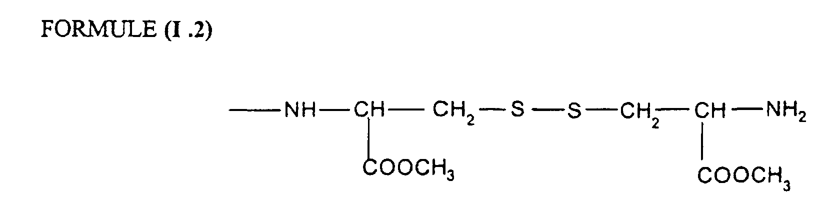

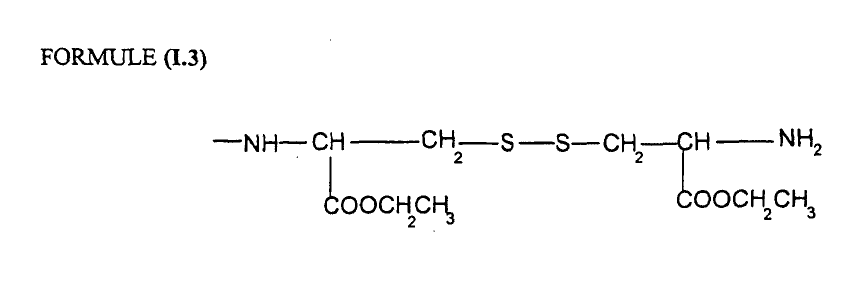

4. Peptide collagénique selon la revendication 2, caractérisé :
◆ en ce qu'il comprend des restes mercaptoaminés greffés de formule (I) telle que définie dans la revendication 2 et dans laquelle R² correspond à l'hydrogène
◆ et en ce qu'il est réticulable.

5. Peptide collagénique selon la revendication 4 **caractérisé en ce qu'**il comprend des restes mercaptoaminés de formule (**I'**) suivante : 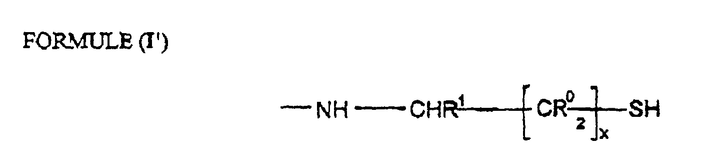 dans laquelle R¹ correspond à H ou à COOR³, avec x, R¹, R⁰, R³, tels que définis supra dans la revendication 2 en légende de la formule (I), R³ pouvant, en outre, représenter l'hydrogène ou un cation apte à former un sel avec COO⁻, ce cation étant, de préférence, Na⁺, K⁺, Li⁺.

6. Peptide collagénique réticulé caractérisé :
• en ce qu'il comprend des chaînes collagéniques reliées entre elles par des ponts disulfure dont les atomes de soufre constitutifs appartiennent à des restes mercaptoaminés exclusivement greffés, sur les acides aspartique et glutamique des chaînes collagéniques, par l'intermédiaire de liaisons amides
• en ce qu'il est obtenu à partir du peptide collagénique selon la revendication 4 et/ou 5.

7. Peptide collagénique selon l'une quelconque des revendications 1 à 6 **caractérisé en ce qu'**il comporte des greffons G, différents des restes mercaptoaminés, (notamment ceux tels que définis supra dans les revendications 1 à 6), fixés sur au moins une partie des motifs amine libres de la chaîne collagénique, par l'intermédiaire de liaisons amides, G étant un acyle comprenant une entité hydrocarbonée, comportant, éventuellement, des hétéroatomes (avantageusement 0 et/ou N) et étant, de préférence, choisie parmi les alkyles et/ou les alcényles et/ou les alicycliques et/ou les aromatique et, plus préférentiellement encore, parmi les groupements comprenant une chaîne alkyle, éventuellement insaturée, comprenant de 1 à 22 carbone(s) ou répondant à la formule (**III**) suivante :
étant, de préférence, choisie parmi les alkyles et/ou les alcényles et/ou les alicycliques et/ou les aromatique et, plus préférentiellement encore, parmi les groupements comprenant une chaîne alkyle, éventuellement insaturée, comprenant de 1 à 22 carbone(s) ou répondant à la formule **(III)** suivante : 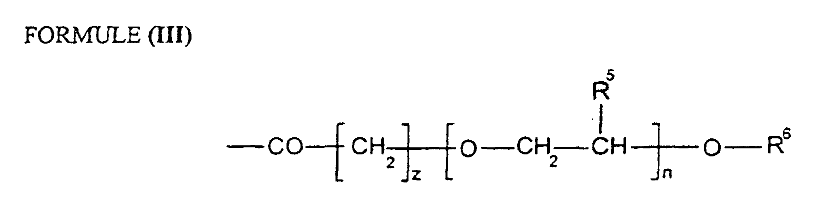 avec :
• R⁵ = H ou CH3;
• R⁶ = H, un radical alkyle linéaire ou ramifié et de préférence un méthyle ;
• z=0,1 ou 2 et n > 0.

8. Procédé d'obtention d'un peptide collagénique **soluble en milieu aqueux et/ou dans les solvants polaires** et modifié par greffage de fonctions thiol substituées et portées par des restes mercaptoaminés,
**caractérisé en ce qu'**il consiste essentiellement à faire réagir en solution le peptide collagénique avec au moins un précurseur d'un reste mercaptoaminé dont la fonction thiol et l'éventuelle fonction carboxylique sont bloquées, en présence d'au moins un agent de greffage, de préférence choisi dans le groupe comprenant les produits activant les groupements carboxyliques, de préférence les carbodiimides.

9. Procédé de préparation d'un peptide collagénique réticulable, modifié par greffage de fonctions thiol libres et portées par des restes mercaptoaminés, **caractérisé en ce qu'**il consiste, essentiellement :
1. à faire réagir en solution le peptide collagénique avec au moins un précurseur d'un reste mercaptoaminé dont la fonction thiol et l'éventuelle fonction carboxylique sont bloquées, en présence d'au moins un agent de greffage, de préférence choisi dans le groupe
2. et à déprotéger (transformation en thiols) les fonction mercapto des Testes mercaptoaminés greffés sur les peptides collagéniques modifiés obtenus à l'étape 1.

10. Procédé de préparation d'un peptide collagénique réticulé à partir d'un peptide collagénique modifié par greffage de fonctions thiol libres et portées par des restes mercaptoaminé, **caractérisé en ce qu'**il consiste, essentiellement :
1. à faire réagir en solution, exclusivement les fonctions carboxyliques des acides aspartique et glutamique d'un peptide collagénique avec au moins un précurseur d'un reste mercaptoaminé dont la fonction thiol et l'éventuelle fonction carboxylique sont bloquées, en présence d'au moins un agent de greffage, de préférence choisi dans le groupe comprenant les produits activant les groupements carboxyliques, de préférence les carbodiimides ;
2. et à déprotéger (transformation en thiols) les fonctions mercapto des restes mercaptoaminés greffés sur les peptides collagéniques modifiés obtenus à l'étape 1 ;
3. et à oxyder les fonctions thiol du peptide collagénique modifié réticulable issu de l'étape 2, de façon à former des ponts disulfure intercaténaires.

11. Procédé selon l'une quelconque des revendications 8 à 10 **caractérisé en ce que** l'on prévoit une étape complémentaire F de fonctionnalisation par des greffons G de nature différente de celle des greffons fixés aux fonctions carboxyliques des acides aspartiques et glutamiques, cette étape F consistant essentiellement à procéder à une acylation d'au moins une partie des fonctions amines libres de la chaîne collagénique, de façon à fixer sur celles-ci des greffons G comprenant une entité hydrocarbonée, cette entité comprenant, éventuellement, des hétéroatomes (avantageusement O et/ou N), et étant, de préférence, choisie parmi les alkyles et/ou les alcényles et/ou les alicyliques et/ou les aromatiques.

12. Application des peptides collagéniques selon l'une quelconque des revendications 1 à 7 ou du peptide obtenu par le procédé selon l'une quelconque des revendications 8 à 11, à titre de biomatériau constitutif d'implants, de prothèses, de pansements, de tissus artificiels, de système de bioencapsulation, de revêtement de biocompatibilisation, de fils de suture, de colles ou de ciments chirurgicaux ou de support pour culture cellulaire.

## Patentansprüche

1. Kollagenpeptid, das durch Pfropfung von freien oder substituierten Thiol-Gruppen, die durch Mercaptoamin-Reste getragen werden, modifiziert ist, **dadurch gekennzeichnet,**
• **daß** diese Mercaptoamin-Reste untereinander identisch oder verschieden sind und ausschließlich auf Aspargin- und Glutaminsäuren der Kollagenkette durch Zwischen-Amidbindungen gepfropft sind,
• und **daß** es in wäßrigem Milieu und/oder in polaren Lösungsmitteln löslich ist.

2. Kollagenpeptid gemäß Anspruch 1, **dadurch gekennzeichnet, daß** mindestens ein Teil der Mercaptoamin-Reste, die auf den Carboxylsäuren der Aspargin- und Glutaminsäuren gepfropft sind, der folgenden allgemeinen Formel (I) entspricht: 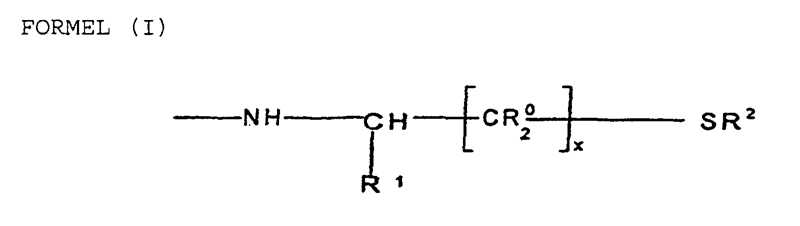 worin:
• x = 1 oder 2;
• R⁰ = H oder CH₃;
• R¹ H oder COOR³ wiedergibt, wobei R³ einem aliphatischen, aromatischen oder alicyklischen, vorzugsweise einem alkylischen, alkenylischen, arylischen, aralkylischen, alkylarylischen oder alkenylarylischen, und noch weiter bevorzugt einem methylischen oder ethylischen Kohlenwasserstoffrest entspricht;
• R² ein aliphatischer und/oder alicyclischer und/oder aromatischer Rest ist, vorzugsweise ein Alkyl oder ein ggf. sulfiertes und/oder aminiertes Acyl, und wobei R² weiter bevorzugt der folgenden Formel (II) entspricht: 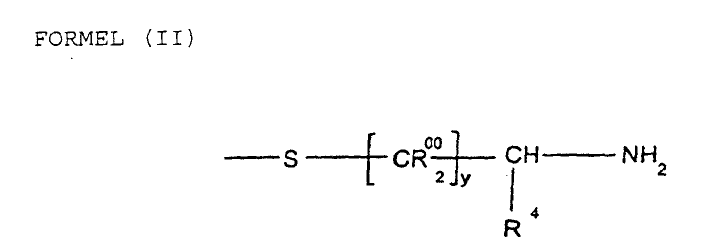
• wobei y, R⁰⁰ und R⁴ der gleichen wie der in der Legende der Formel (I) für y, R⁰ und R¹ gegebenen Definition entspricht.

3. Kollagenpeptid gemäß Anspruch 2, **dadurch gekennzeichnet, daß** die gepfropften Mercaptoamin-Reste aus der Gruppe der folgenden Reste ausgewählt sind:

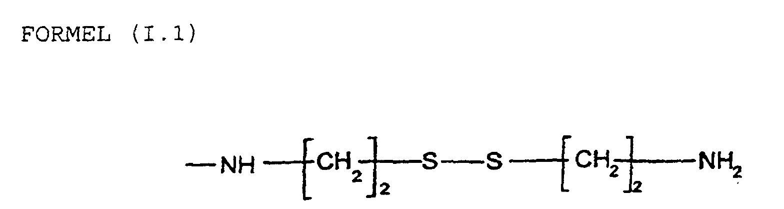

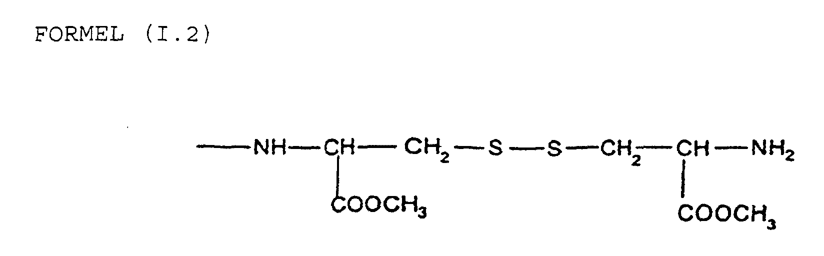

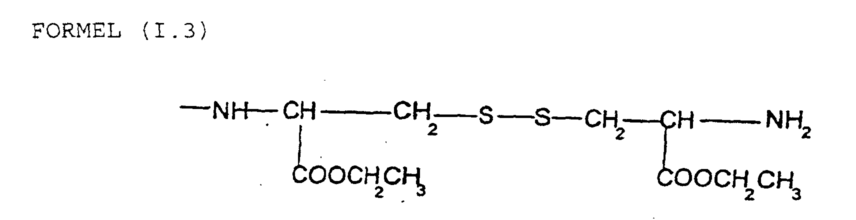

4. Kollagenpeptid gemäß Anspruch 2, **dadurch gekennzeichnet,**
• **daß** es gepfropfte Mercaptoamin-Reste der Formel (I) umfaßt, wie sie im Anspruch 2 definiert sind und worin R² Wasserstoff entspricht
• und **daß** es vernetzbar ist.

5. Kollagenpeptid gemäß Anspruch 4, **dadurch gekennzeichnet, daß** es Mercaptoamin-Reste der folgenden Formel (I') umfaßt: 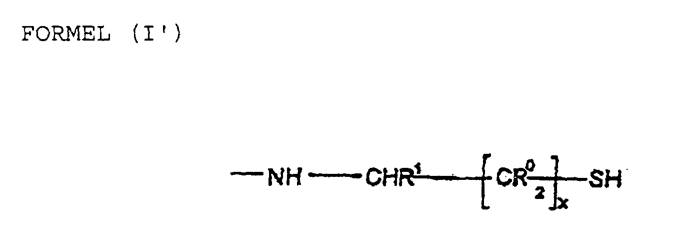 worin R¹ H oder COOR³ entspricht, wobei x, R¹, R⁰, R³ wie oben im Anspruch 2 in der Legende der Formel (I) definiert sind, wobei R³ andererseits auch Wasserstoff oder ein Kation, das zur Bildung eines Salzes mit COO⁻ geeignet ist, wiedergeben kann, wobei dieses Kation vorzugsweise Na⁺, K⁺ oder Li⁺ ist.

6. Vernetztes Kollagenpeptid, **dadurch gekennzeichnet,**
• **daß** es Kollagenketten umfaßt, die untereinander durch Disulfidbrücken vernetzt sind, deren aufbauende Schwefelatome zu Mercaptoamin-Resten gehören, die ausschließlich durch Zwischen-Amidbindungen auf Aspargin- und Glutaminsäuren der Kollagenketten gepfropft sind,
• **daß** es, ausgehend vom Kollagenpeptid gemäß Anspruch 4 und/oder 5 erhalten wurde.

7. Kollagenpeptid gemäß irgendeinem der Ansprüche 1 bis 6, **dadurch gekennzeichnet, daß** es Pfropfteile G, die sich von den Mercaptoamin-Resten (insbesondere jenen, die oben durch die Ansprüche 1 bis 6 definiert sind) unterscheiden, umfaßt, die durch Zwischen-Amidverbindungen mit mindestens einem Teil der freien Aminstruktureinheiten der Kollagenkette verbunden sind, wobei G ein Acyl ist, das Kohlenwasserstoffeinheit umfaßt, die ggf. Heteroatome (vorteilhafterweise O und/oder N) trägt und vorzugsweise ausgewählt ist unter Alkyl und/oder Alkenyl und/oder Alicyclen und/oder Aromaten und weiter bevorzugt unter Gruppen, die eine ggf. ungesättigte, 1 bis 22 Kohlenstoffatome umfassende Alkylkette umfassen, oder der folgenden Formel (III) entspricht: 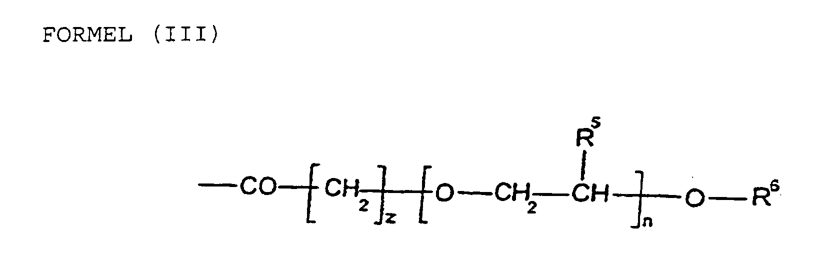 wobei:
• R⁵ = H oder CH₃;
• R⁶ = H, ein linearer oder verzweigter Alkylrest und vorzugsweise ein Methylrest;
• z = 0, 1 oder 2 und n > 0.

8. Verfahren zum Erhalt eines Kollagenpeptids, das in wäßrigem Milieu und/oder in polaren Lösungsmitteln löslich ist und durch Pfropfung von Thiol-Gruppen modifiziert ist, die substituiert sind und durch Mercaptoamin-Reste getragen werden,
**dadurch gekennzeichnet, daß** es im wesentlichen aus einem Reagierenlassen des Kollagenpeptids in Lösung mit mindestens einem Vorläufer eines Mercaptoamin-Rests, dessen Thiol-Gruppe und ggf. Carboxylgruppe blockiert sind, in Gegenwart von mindestens einem Pfropfungsmittel besteht, welches vorzugsweise aus der Gruppe ausgewählt ist, die Carboxylgruppen-aktivierende Produkte, vorzugsweise Carbodiimide, umfaßt.

9. Verfahren zur Herstellung eines vernetzbaren Kollagenpeptids, das durch Pfropfung von freien und durch Mercaptoamin-Reste getragene Thiol-Gruppen modifiziert ist, **dadurch gekennzeichnet, daß** es im wesentlichen besteht aus:
1. Reagierenlassen des Kollagenpeptids in Lösung mit mindestens einem Vorläufer eines Mercaptoamin-Rests, dessen Thiol-Gruppe und ggf. Carboxylgruppe blockiert sind, in Gegenwart von mindestens einem Pfropfungsmittel, das vorzugsweise ausgewählt wird aus der Gruppe, die Carboxylgruppen-aktivierende Produkte, vorzugsweise Carbodiimide, umfaßt,
2. und Entschützen (Überführung in Thiole) der Mercaptogruppe der Mercaptoamin-Reste, die auf die aus Schritt 1 erhaltenen, modifizierten Kollagenpeptide gepfropft sind.

10. Verfahren zur Herstellung eines vernetzten Kollagenpeptids, ausgehend von einem Kollagenpeptid, das durch Pfropfung von freien und durch Mercaptoamin-Reste getragenen Thiol-Gruppen modifiziert ist, **dadurch gekennzeichnet, daß** es im wesentlichen besteht aus:
1. Reagierenlassen ausschließlich der Carboxylgruppen der Aspargin- und Glutaminsäuren eines Kollagenpeptids in Lösung mit mindestens einem Vorläufer eines Mercaptoamin-Rests, dessen Thiol-Gruppe und ggf. Carboxylgruppe blockiert sind, in Gegenwart von mindestens einem Pfropfungsmittel, das vorzugsweise ausgewählt wurde aus der Gruppe, die Carboxylgruppen-aktivierende Produkte, vorzugsweise Carbodiimide, umfaßt;
2. und Entschützen (Umwandeln in Thiole) der Mercaptogruppen der Mercaptoamin-Reste, die auf die aus Schritt 1 erhaltenen, modifizierten Kollagenpeptide gepfropft sind;
3. und Oxidieren der Thiol-Gruppen des aus Schritt 2 stammenden, vernetzbaren, modifizierten Kollagenpeptids, um Zwischenketten-Disulfidbrücken zu bilden.

11. Verfahren gemäß irgendeinem der Ansprüche 8 bis 10, **dadurch gekennzeichnet, daß** man einen Ergänzungsschritt F der Funktionalisierung durch Pfropfteile G vorsieht, die von jenen der Pfropfteile verschieden sind, die mit den Carboxylgruppen der Aspargin- und Glutaminsäuren verbunden sind, wobei dieser Schritt F im wesentlichen besteht aus der Prozessierung einer Acylierung mindestens eines Teils der freien Amingruppen der Kollagenkette, um daran eine Kohlenwasserstoffeinheit umfassende Pfropfteile G zu binden, wobei diese Einheit ggf. Heteroatome (vorteilhafterweise O und/oder N) umfaßt und vorzugsweise ausgewählt wird unter Alkyl und/oder Alkenyl und/oder Alicyclen und/oder Aromaten.

12. Verwendung der Kollagenpeptide gemäß irgendeinem der Ansprüche 1 bis 7 oder des durch das Verfahren gemäß irgendeinem der Ansprüche 8 bis 11 erhaltenen Peptids als Biokonstruktionsimplantate, Prothesen, Verbände, künstliche Gewebe, Bioverkapselungssystem, Beschichtung zur Biokompatibilisierung, Nähfäden, chirurgische Kleber oder Zemente oder als Träger für die Zellkultur.

## Claims

1. Collagen peptide modified by the grafting of free or substituted thiol groups carried by mercaptoamino radicals, **characterized in that**:
• these mercaptoamino radicals are identical to or different from one another and are exclusively grafted to the aspartic and glutamic acids of the collagen chain via amide linkages, and
• said collagen peptide is soluble in aqueous media and/or in polar solvents.

2. Collagen peptide according to claim 1, **characterized in that** at least some of the mercaptoamino radicals grafted to the carboxylic acids of the aspartic and glutamic acids have general formula **(I)** below: 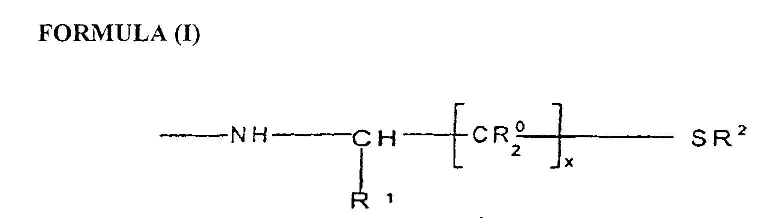 in which:
• x = 1 or 2;
• R⁰ = H or CH₃;
• R¹ is H or COOR³, where R³ is a hydrocarbon radical of aliphatic, aromatic or alicyclic type, preferably an alkyl, alkenyl, aryl, aralkyl, alkylaryl or alkenylaryl radical and particularly preferably a methyl or ethyl radical; and
• R² is an aliphatic and/or alicyclic and/or aromatic radical, preferably an alkyl or acyl optionally containing sulfur and/or amino, and particularly preferably a radical of formula **(II)** below: 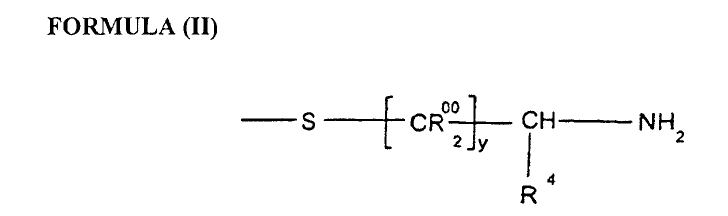
• where y, R⁰⁰ and R⁴ are defined in the same way as x, R⁰ and R¹ in formula (I).

3. Collagen peptide according to claim 2, **characterized in that** the grafted mercaptoamino radicals are selected from the following group of radicals:

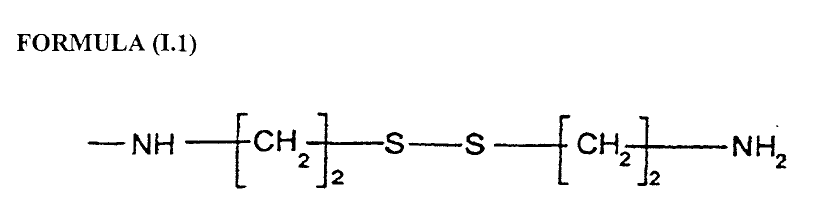

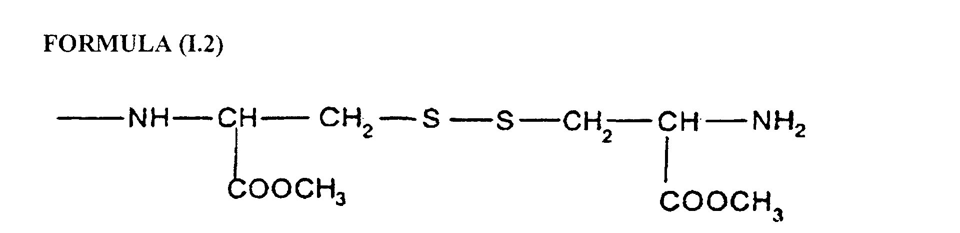

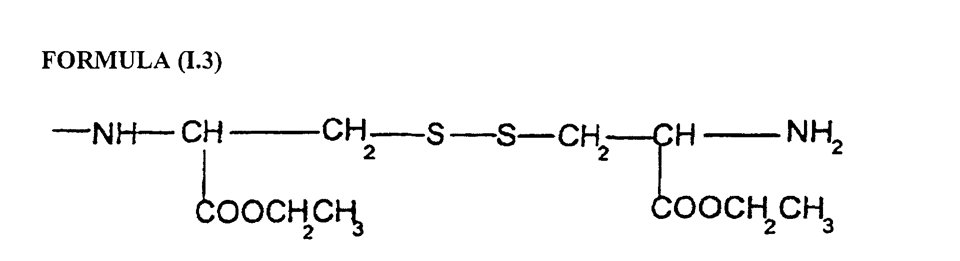

4. Collagen peptide according to claim 2, **characterized in that**:
◆ it comprises grafted mercaptoamino radicals of formula (I) as defined in claim 2 in which R² is hydrogen, and
◆ it is crosslinkable.

5. Collagen peptide according to claim 4, **characterized in that** it comprises mercaptoamino radicals of formula **(I')** below: 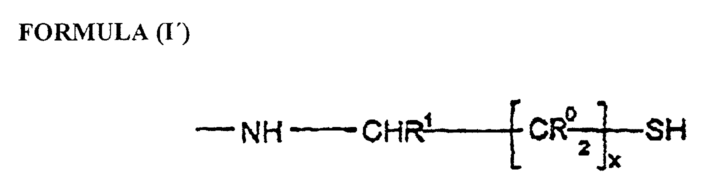 in which R¹ is H or COOR³ and x, R¹, R⁰ and R³ are as defined above for formula (I) in claim 2, it also being possible for R³ to be hydrogen or a cation capable of forming a salt with COO⁻, said cation preferably being Na⁺, K⁺ or Li⁺.

6. Crosslinked collagen peptide, **characterized in that**:
• it comprises collagen chains joined together by disulfide bridges of which the constituent sulfur atoms belong to mercaptoamino radicals exclusively grafted to the aspartic and glutamic acids of the collagen chains via amide linkages, and
• it is obtained from the collagen peptide according to claim 4 and/or 5.

7. Collagen peptide according to any one of claims 1 to 6, **characterized in that** it contains grafts G, different from the mercaptoamino radicals (especially those defined as in claims 1 to 6 above), fixed to at least some of the free amino units of the collagen chain via amide linkages, G being an acyl comprising a hydrocarbon entity optionally containing heteroatoms (advantageously O and/or N) and being selected preferably from alkyls and/or alkenyls and/or alicyclics and/or aromatics and particularly preferably from groups comprising an optionally unsaturated alkyl chain having from 1 to 22 carbons, or having formula **(III)** below:
selected preferably from alkyls and/or alkenyls and/or alicyclics and/or aromatics and particularly preferably from groups comprising an optionally unsaturated alkyl chain having from 1 to 22 carbons, or having formula **(III)** below: 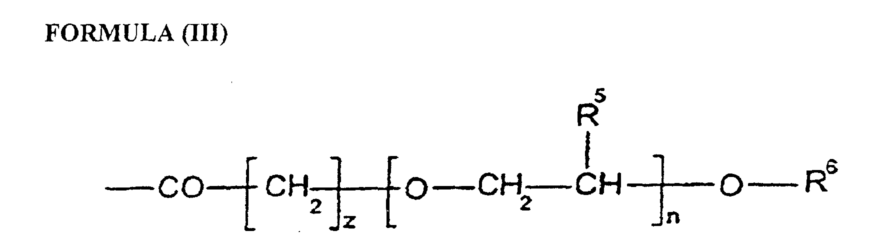 where:
• R⁵ = H or CH₃;
• R⁶ = H or a linear or branched alkyl radical, preferably a methyl; and
• z = 0, 1 or 2 and n > 0.

8. Process for the preparation of a collagen peptide **soluble in aqueous media and/or in polar solvents** and modified by the grafting of substituted thiol groups carried by mercaptoamino radicals, **characterized in that** it consists essentially in reacting the collagen peptide in solution with at least one precursor of a mercaptoamino radical in which the thiol group and the carboxyl group, if present, are blocked, in the presence of at least one grafting agent preferably selected from the group comprising products that activate carboxyl groups, preferably carbodiimides.

9. Process for the preparation of a crosslinkable collagen peptide modified by the grafting of free thiol groups carried by mercaptoamino radicals, **characterized in that** it consists essentially in:
1. reacting the collagen peptide in solution with at least one precursor of a mercaptoamino radical in which the thiol group and the carboxyl group, if present, are blocked, in the presence of at least one grafting agent preferably selected from the group ...
2. and deprotecting (converting to thiols) the mercapto groups of the mercaptoamino radicals grafted to the modified collagen peptides obtained in step 1.

10. Process for the preparation of a crosslinked collagen peptide from a collagen peptide modified by the grafting of free thiol groups carried by mercaptoamino radicals, **characterized in that** it consists essentially in:
1. reacting, in solution, exclusively the carboxyl groups of the aspartic and glutamic acids of a collagen peptide with at least one precursor of a mercaptoamino radical in which the thiol group and the carboxyl group, if present, are blocked, in the presence of at least one grafting agent preferably selected from the group comprising products that activate carboxyl groups, preferably carbodiimides;
2. deprotecting (converting to thiols) the mercapto groups of the mercaptoamino radicals grafted to the modified collagen peptides obtained in step 1; and
3. oxidizing the thiol groups of the crosslinkable modified collagen peptide obtained in step 2 in order to form intercatenary disulfide bridges.

11. Process according to any one of claims 8 to 10, **characterized in that** provision is made for a complementary step F for functionalization with grafts G different in nature from the grafts fixed to the carboxyl groups of the aspartic and glutamic acids, said step F consisting essentially in acylating at least some of the free amino groups of the collagen chain in order to fix thereto grafts G comprising a hydrocarbon entity, said entity optionally comprising heteroatoms (advantageously O and/or N) and preferably being selected from alkyls and/or alkenyls and/or alicyclics and/or aromatics.

12. Application of the collagen peptides according to any one of claims 1 to 7 or of the peptide obtained by the process according to any one of claims 8 to 11 as a constituent biomaterial of implants, prostheses, dressings, artificial tissues, bioencapsulation systems, biocompatibilization coverings, suture threads, surgical glues or cements, or cell culture substrates.
